# EUROPÄISCHE PATENTANMELDUNG

(11) **EP 2 857 056 A1**
(43) Veröffentlichungstag der Anmeldung: **08.04.2015**
(21) Anmeldenummer: 14003352.3
(22) Anmeldetag: 29.09.2014
(51) Int. Cl.: A61M 21/00, F21V 33/00, F21V 3/00, F21Y 105/00, F21Y 111/00

(54) **Entspannungsraum im Deepsea-Konzept**

(30) Priorität: 01.10.2013 CH 16812013
(71) Anmelder: Sonami AG, 9490 Vaduz (LI)
(72) Erfinder: Lathan, Daniel, 9490 Vaduz (LI)

(57) **Zusammenfassung**

Ein Entspannungsraum (1) mit zur Erzeugung verschiedenartiger Reize eines Menschen (2) ausgebildeten Einrichtungen (3) sowie mit einem begehbaren Innenraum (4), wobei der Innenraum (4) vollständig abdunkelbar sowie an seinen Randflächen (5) mit dreidimensional verteilten Lichteffekten punktuell beleuchtbar ist.

## Beschreibung

Die Erfindung betrifft einen von mehreren Menschen begehbaren Entspannungsraum, der im Innenraum mittels geeigneter Einrichtungen unübliche Empfindungen hervorruft, welche die Sinne des Menschen auf eine Art und Weise ansprechen, die Entspannungs- und Entstressungsvorgänge unterstützt. Nach der DE19814499C2 ist eine Wohlfühl-Sauna für den Privatbereich bekannt, die optische, olfaktorische, akustische und taktile Reize des Menschen anspricht. Die Aufgabe der Erfindung besteht in einer auch zur Verwendung im professionellen Wohlfühl-Bereich geeigneten Ausführung eines begehbaren Entspannungsraums. Die Aufgabe wird mit den Merkmalen des Anspruchs 1 gelöst. Weiterbildungen ergeben sich aus den Unteransprüchen.

Im Wesentlichen weist ein Entspannungsraum, welcher Einrichtungen zur Erzeugung verschiedenartiger Reize des Menschen aufweist, einen von mehreren Menschen begehbaren Innenraum auf, welcher vollständig abdunkelbar sowie an seinen Randflächen mit dreidimensional verteilten Lichteffekten punktuell beleuchtbar ist.

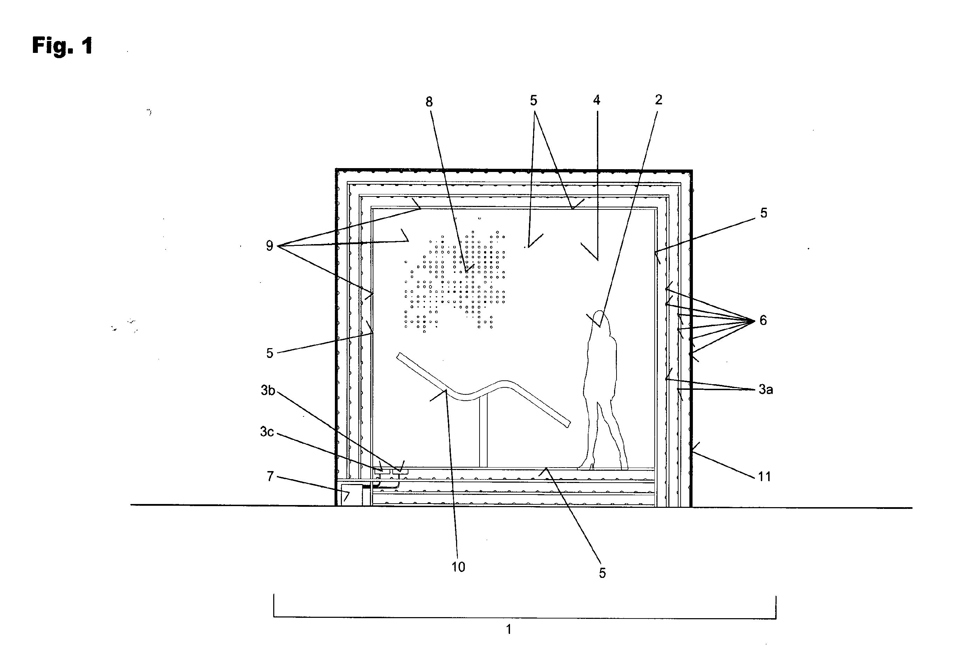

Das nachfolgende, anhand der geschnittenen Fig. 1 erläuterte, Ausführungsbeispiel kombiniert sämtliche weiteren, optionalen Merkmale. So weist ein Entspannungsraum 1 zur Erzeugung verschiedenartiger Reize eines Menschen 2 ausgebildete Einrichtungen 3 sowie einen von mehreren Menschen 2 durch ein nicht dargestelltes, lichtdicht verschliessbares Labyrinth begehbaren quaderförmigen Innenraum 4 auf, welcher an jeder seiner Randflächen 5 jeweils mehrere (hier drei) in deren Tiefe versetzte Leuchtschichten 6 mit matrixförmig verteilten punktuellen Lichtstrahlern 3a in Form von RGB-LED Elementen aufweist, welche jeweils mit einem Steuergerät 7 verbunden einzeln ansteuerbar sind. Die äussere Leuchtschicht 6 ist aussenseitig lichtdicht abgedunkelt. Insbesondere können damit programmgesteuert diffuse Leuchteffekte aus der Tiefsee inspiriert werden. Die Lichtstrahler 3a jeder Leuchtschicht 6 sind innenseitig auf einer Sichtfläche 9 aus transluzenten Material in Form von milchigem Plexiglas montiert, wodurch ausser der inneren jeder Leuchtschicht 6 zum Innenraum 4 hin eine Sichtfläche 9 der benachbarten Leuchtschicht 6 zugeordnet ist. Die zusätzlichen innersten Sichtflächen 8 bilden zudem tönende und vibrierende Flächen von rückseitigen Körperschallwandlern (Excitern) 3b und Aktoren 3c aus, die jeweils mit dem Steuergerät 7 verbunden einzeln ansteuerbar sind. Insbesondere können damit reale Geräusche und Vibrationen der Tiefsee wiedergegeben sowie auf Liegemöbel 10 in Form von Liegen übertragen werden. Dieser Entspannungsraum findet im professionellen Wohlfühl-Bereich kombiniert mit Entspannungs- und Stressmanagement-Methoden wie Massagen, Gymnastik, autogenes Training, Meditation, Kuren, Therapiegesprächen etc. Anwendung.

## Patentansprüche

1. Entspannungsraum mit zur Erzeugung verschiedenartiger Reize eines Menschen (2) ausgebildeten Einrichtungen (3) sowie mit einem begehbaren Innenraum (4), **dadurch gekennzeichnet, dass** der Innenraum (4) vollständig abdunkelbar sowie an seinen Randflächen (5) mit dreidimensional verteilten Lichteffekten punktuell beleuchtbar ist.

2. Entspannungsraum nach Anspruch 1, **dadurch gekennzeichnet, dass** an ebenen Randflächen (5) jeweils mehrere in deren Tiefe versetzte Leuchtschichten (6) mit matrixförmig verteilten punktuellen Lichtstrahlern (3a) vorhanden sind, welche jeweils mit einem Steuergerät (7) verbunden einzeln ansteuerbar sind.

3. Entspannungsraum nach Anspruch 2, **dadurch gekennzeichnet, dass** mit dem Steuergerät (7) programmgesteuert Leuchteffekte aus der Tiefsee inspirierbar sind.

4. Entspannungsraum nach einem der Ansprüche 2 bis 3, **dadurch gekennzeichnet, dass** an jeder Randseite (5) jede Leuchtschicht (6) zum Innenraum (4) hin eine Sichtfläche (9) aus transluzentem Material aufweist, welche mit Lichtstrahlern (3a) beleuchtbar ist.

5. Entspannungsraum nach Anspruch 4, **dadurch gekennzeichnet, dass** die innersten Sichtflächen (8) tönende und/oder vibrierende Flächen von Körperschallwandlern (3b) und/oder Aktoren (3c) ausbilden, die jeweils mit dem Steuergerät (7) verbunden einzeln ansteuerbar sind.

6. Entspannungsraum nach einem der Ansprüche 1 bis 5, **dadurch gekennzeichnet, dass** mit den Körperschallwandlern (3b) und/oder Aktoren (3c) reale Geräusche und/oder Vibrationen der Tiefsee erzeugbar sowie auf Liegemöbel (10) übertragbar sind.

7. Verwendung eines Entspannungsraums nach einem der Ansprüche 1 bis 6 im professionellen Wohlfühl-Bereich.
